# EUROPEAN PATENT SPECIFICATION

(11) **EP 1 997 444 B1**
(45) Date of publication and mention of the grant of the patent: **14.09.2011**
(21) Application number: 08251759.0
(22) Date of filing: 20.05.2008
(51) Int. Cl.: A61B 17/34, F16J 15/32, A61B 17/32

(54) **Access assembly with whisker seal**
Zugriffsanordnung mit Whiskerabdichtung
Ensemble d'accès avec joint de trichite

(30) Priority: 22.05.2007 US 931254 P
(43) Date of publication of application: 03.12.2008
(73) Proprietor: Tyco Healthcare Group LP, North Haven, CT 06473 (US)
(72) Inventor: Brockmeier, Oivind, Somerville, MA 02144 (US); Focht, Kenneth Allen, Needham, MA 02492 (US); Judson, Jared Alden, Topsfield, MA 01983 (US)
(74) Representative: Soames, Candida Jane

(56) References cited:
- US-A- 4 779 904
- US-A- 5 480 165
- US-A- 5 743 884
- US-A- 5 997 515
- US-A1- 2004 092 862

## Description

### BACKGROUND

### 1. Technical Field

The present disclosure relates to an access apparatus for providing access to an underlying surgical site, and, more particularly, relates to an access apparatus incorporating a novel seal mechanism adapted to permit ease of insertion of a surgical instrument while providing a substantial seal about the instrument upon manipulation during the surgical procedure.

### 2. Background of Related Art

Surgical access apparatii are employed in various minimally invasive procedures including laparoscopic or endoscopic procedures. Such access apparatii are inclusive of portals, trocar cannulas, catheters, or, in the event of a minimally invasive hand assist procedures, hand access devices. Surgical access apparatii typically incorporate a seal mechanism to form a fluid tight seal about an instrument or hand passed through the portal. The seal mechanisms, however, often are limited, in part, due to the large insertion forces required to pass the object through the seal of the seal mechanism. In addition, off-axis movement of the instrument within the seal may be too difficult or easy depending on the type of seal employed within the portal. Moreover, the seal mechanisms are also limited by their ability to sustain their integrity when the surgical instrument is angulated. Such extreme ranges of motion of smaller diameter surgical instruments within the portal can create a "cat eye" or crescent shaped gap about the instrument resulting in fluid loss (e.g., insufflation gas loss). Document US-A-2004/0092862 discloses a surgical access assembly having the features of the preamble of claim 1.

### SUMMARY

Accordingly, the present disclosure is directed to a surgical access assembly for sealed reception of an elongated object as claimed in claim 1. The surgical access assembly includes an access member having at least one opening configured and dimensioned to permit entry of an elongated object and being adapted for positioning within tissue to provide access to an underlying surgical site. A seal member is mounted relative to the access member. The seal member includes an outer substrate and a plurality of flexible bristle members extending radially inwardly from the outer substrate to the central longitudinal axis. The bristle members define free ends remote from the outer substrate and being adapted to flex and form a substantial seal about the elongated object. The fixe ends of the bristle members have an axial component of direction w.r.t. the central longitudinal axis in the absence of the elongated object. The bristle members can be dimensioned to define a thickness or density which is greater at the free ends thereof relative to remaining portions of the bristle members. Other preferred embodiment, are disclosed in the dependent claims.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.
FIG. 1 is a perspective view with parts separated of an access apparatus in accordance with the principles of the present disclosure, illustrating a cannula assembly, an obturator assembly positionable within the cannula assembly and a seal assembly;
FIG. 2 is an enlarged perspective view with parts separated of the seal assembly of FIG. 1 illustrating the seal housing and the seal member;
FIG. 3A is an enlarged perspective view of a flat brush profile for use as a seal member;
FIG. 3B is an enlarged perspective view of a seal member formed with the flat brush profile of FIG. 3A;
FIG. 4 is a side elevational view of an embodiment of the invention of the seal member of FIGS. 3A-3B;
FIGS. 5-6 depict alternative brush profiles which may be used to form a seal member;
FIG. 7A is a perspective view of an alternate example of a seal member having a step brush profile;
FIG. 7B is a cutaway perspective view of the seal member of FIG. 7A;
FIG. 8 is a side plan view in partial cross section of the access apparatus of FIGS. 7A-7B;
FIG. 9 is an enlarged, partial cross-sectional view of the area of detail indicated in FIG. 8 illustrating a surgical instrument inserted through the seal assembly;
FIG. 10 is a view similar to FIG. 9 illustrating the adaptability of the seal assembly to variously sized surgical instruments and radial movement of those instruments; and
FIGS. 11-14 are views illustrating alternate example of seal assemblies of the present disclosure.

### DETAILED DESCRIPTION OF PREFERRED EMBODIMENTS

In the drawings and in the description which follows, the term "proximal", as is traditional, will refer to the end of the apparatus which is closest to the clinician, while the term "distal" will refer to the end which is furthest from the clinician.

The present disclosure contemplates the introduction into a body cavity of all types of surgical instruments including clip appliers, graspers, dissectors, retractors, staplers, laser fibers, photographic devices, endoscopes and laparoscopss, tubes, and the like. All such objects are referred to herein as "instrument(s)".

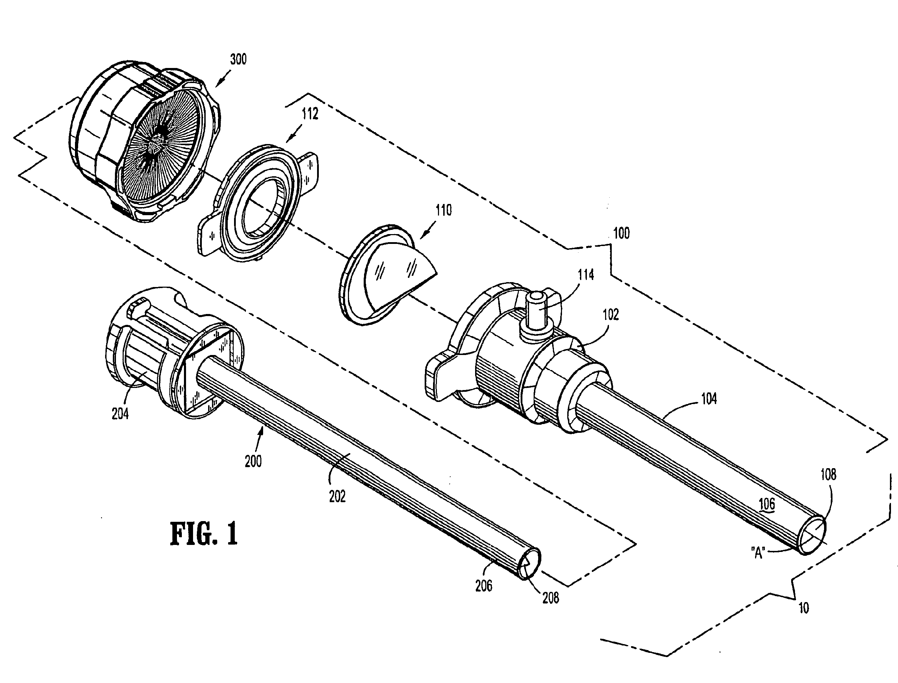

Referring now in detail to the drawing figures, in which like references numerals identify similar or identical elements, there is illustrated, in FIG. 1, a surgical system in accordance with the present disclosure. System 10 has particular application in laparoscopic procedures with respect to accessing the abdominal cavity, and the like, and may be used in any such surgical procedure where the peritoneal cavity is insufflated with a suitable gas, e.g., CO₂, to separate the cavity wall from the internal organs housed therein. System 10 includes cannula assembly 100 and obturator assembly 200, which is positionable therein.

Obturator assembly 200 includes obturator 202, which includes obturator housing 204 and sleeve or outer member 206 extending therefrom. Obturator housing 204 is advantageously dimensioned for grasping by a clinician. Obturator 202 further includes penetrating member 208 within sleeve 206. Penetrating member 208 punctures the abdominal cavity or the like, thereby creating an access point through which at least a portion of a surgical procedure may be conducted. Sleeve 206 may be adapted to retract upon insertion into tissue to expose penetrating member 208 to permit the penetrating member 208 to incise the tissue. Alternatively, penetrating member 208 may be adapted to advance within sleeve 206. Following penetration, obturator assembly 200 is removed from cannula assembly 100 to permit the subsequent introduction of surgical instrumentation utilized to carry out the remainder of the procedure through cannula assembly 100.

Referring still to FIG. 1, cannula assembly 100 will be discussed. In one embodiment, cannula assembly 100 includes cannula housing 102 and cannula member 104 having an outer wall 106 and defining longitudinal axis "A". Cannula member 104 defines an internal longitudinal lumen 108 within outer wall 106 dimensioned to permit the passage of surgical instrumentation therethrough. Either or both of cannula housing 102 and cannula member 104 may be opaque or transparent, either wholly or in part, and may be fabricated from any biocompatible material including metals or polymers. Cannula housing 102 also incorporates cannula valve 110, and stabilizing plate 112 which secures the cannula valve 110 to cannula housing 102. Cannula valve 110 is a zero closure valve adapted to assume a substantially closed position in the absence of an instrument to prevent passage of gases therethrough. In one embodiment, cannula valve 110 is a duckbill or trumpet valve. Cannula housing 102 further includes stop cock valve 114 which is connectable to a source of insufflation fluids to distribute the fluids to the underlying body cavity.

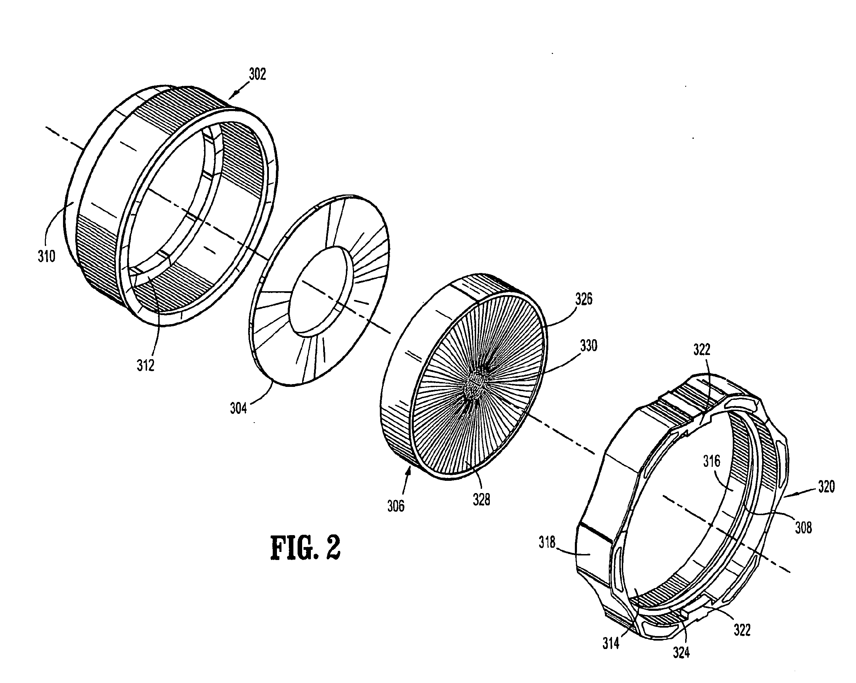

Referring now to FIG. 2 in conjunction with FIG. 1, instrument seal assembly 300 of the present disclosure is adapted to provide a substantial seal between a body cavity of a patient and the atmosphere outside the patient while an instrument is inserted through the cannula assembly 100. Seal assembly 300 may be formed integrally with cannula assembly 200, or may preferably be detachably mounted to cannula assembly 200. Additionally, seal assembly 300 may be readily adapted to be mounted to conventional cannulas of differing structures. Among other advantages, the detachability of seal assembly 300 facilitates the removal of irregularly shaped body tissue and reduces the profile of the cannula whenever the seal assembly is unnecessary for a portion of the surgical procedure.

Seal assembly 300 includes end cap 302, stabilizer plate 304, seal member 306 and seal housing 308. End cap 302, stabilizer plate 304 and seal housing 308 form the outer seal body of seal assembly 100, which houses the sealing component, i.e., seal member 306. End cap 302 is generally cylindrically-shaped and includes proximal end portion 310 defining a diameter which is less than the diameter of the remaining portion of the end cap 302, and an inner peripheral ledge 312 which supports stabilizer plate 304. Seal housing 308 includes central opening 314, inner cylindrical portion 316 and distal outer flange 318 having a scalloped surface to facilitate handling thereof. Cylindrical portion 316 is received within end cap 302 when the seal assembly 300 is fully assembled to enclose the sealing components. Seal housing 308 includes peripheral groove 320 and two opposed ribs 322 extending radially inwardly adjacent the groove 320. Groove 320 and ribs 322 assist in mounting seal assembly 300 to cannula assembly 200 as will be appreciated from the description provided below. Seal housing 308 also includes second groove 324 adjacent opening 314 for accommodating seal member 306.

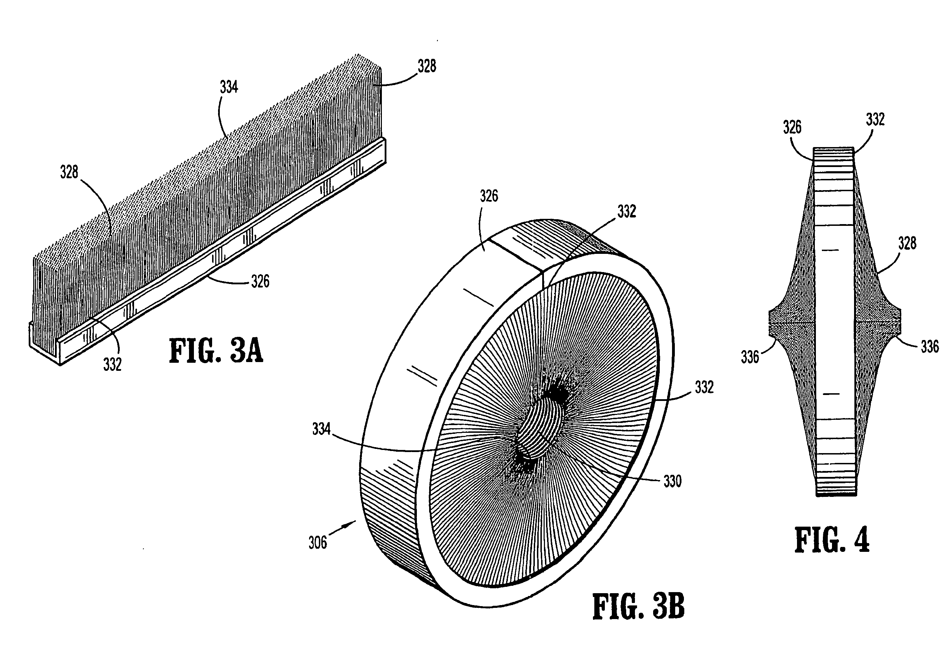

Referring now to FIGS. 2 and 3A-3C, seal member 306 includes substrate 326 and a plurality of bristle members 328 attached to the substrate 326 and extending therefrom. Bristle members 328 define aperture 330, which as will be described in greater detail below, need not be dimensioned uniformly through the seal member 306. As best depicted in FIG. 3A, substrate 326 may be formed as a u-shaped channel such that a plurality of perpendicularly projecting bristle members 328 may be clamped therein by crimping the ends of the u-shaped channel. Alternatively, substrate 326 may be a strip of adhesive material, an overmolded element molded onto one end of bristle members 328 or the like. Each bristle member 328 thus will have a secured end 332 adjacent to substrate 326 and a free end 334 opposite the secured end 332. Of course, other similar methods may be used to provide a generally flat substrate with bristle members 328 projecting perpendicularly from a single face of the substrate. This arrangement defines a flat brush profile wherein all bristle members are generally the same length. A generally flat surface is formed by the free ends 334 of the bristle members 328 being generally parallel to substrate 326.

Bristle members 328 are preferably fabricated from an elastomeric material such as synthetic or natural rubber which is preferably sufficiently resilient to accommodate and provide a substantial seal with instruments of varying diameters. The geometry of bristle members 328 is preferably long and slender.

Flat substrate 326 is curved to form a generally round ring structure to provide seal member 306 generally as seen in FIG. 3B. With this arrangement of substrate 326, bristle member 328 are directed radially inwardly to define aperture 330 with free ends 334 of the bristle members 328 in close proximity with each other as compared to secured ends 332 of the bristle members 328 which substantially maintain their spacing near the substrate 326. This congregation of free ends 334 of bristle member 328 can result in a higher bristle density at the center of the ring near aperture 330 than at the outer regions near substrate 326. If the number and geometry of bristle member 328 is sufficient, a saturation point may be reached where free ends 334 of the bristle member 328 require more space than is available within the confines of the cylinder defined by the substrate 326 thereby resulting in opposed bulges 336 formed near the center of the ring as can be seen in the alternate embodiment of FIG. 4 whereby free ends 334 have an axial component of direction corresponding to the invention as claimed. Seal member 306 arranged in this manner can provide robust lateral support for relatively small instruments having outer dimensions approximating the diameter of aperture 330.

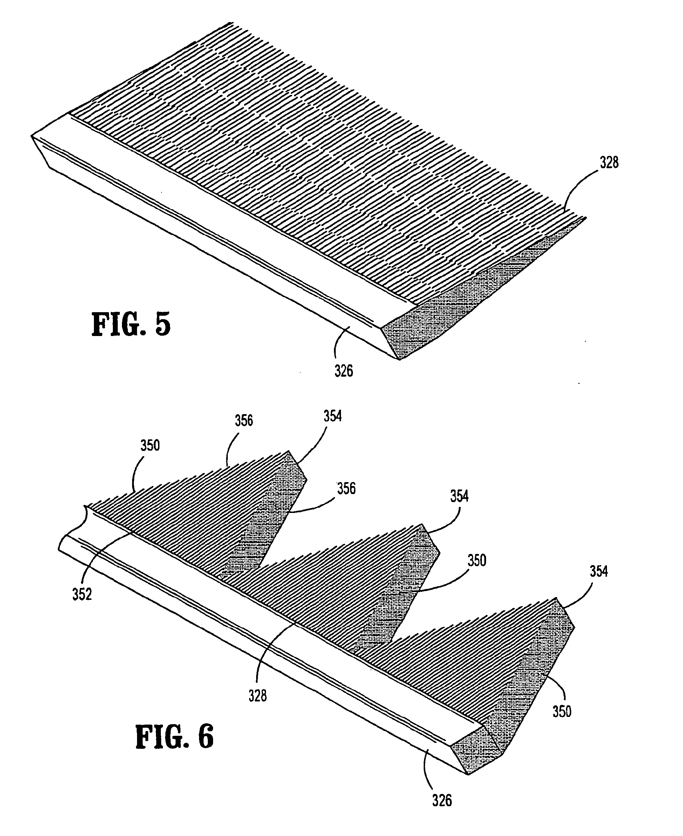

To provide for a decreased insertion force, particularly for larger instruments, several alternate brush profiles may be considered. For example, a tapered brush profile, as shown in FIG. 5, can produce a seal member having a lower required insertion force for larger instruments. This is because fewer bristles will need to compete for the limited space near the aperture 330, and fewer bristles will need to bend to accommodate the instrument. This arrangement is characterized by a brush profile which is uniform along the length of the substrate 326 with a decreasing bristle cross-sectional dimension in the direction of the free ends of the bristles. Depending on the decrease in bristle cross-section, when this profile is formed into a ring, any bulge created in the case of the straight brush profile may be reduced or eliminated resulting in a relatively thin seal member.

Alternatively, a saw-tooth brush profile as shown in FIG. 6 may be provided. The saw-tooth profile is characterized by bristle members 328 arranged in groups or sets 350 shaped to form a triangular arrangement along the length of the substrate 130. Each series 350 has a base 352 formed by the secured ends of bristle members 328, vertex 354, and two sides 356 formed by the free ends of the bristle members 328. As the substrate 326 is formed into a round ring shape, the triangular teeth will begin to converge as the bristles begin to compete for space. Only the longest bristles forming vertex 354 will reach the center of the ring to define the aperture of the seal thereby resulting in a reduced bristle density at the center of the ring. At several internal diameters of the ring, the free ends of the bristles forming the sides of the triangular arrangement will tend to congregate forming multiple bands where relatively robust lateral support will be provided.

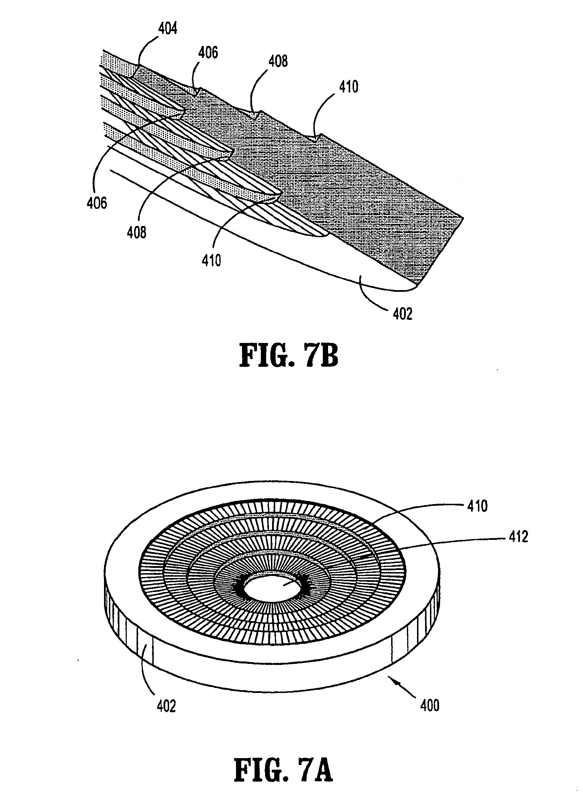

Referring now to FIGS. 7A and 7B, a stepped brush profile may be provided to create interior rings of high bristle density at the free ends of the bristles and corresponding lateral stiffness. This profile is characterized by bristles of several discrete lengths protruding from the substrate and arranged in distinct steps. The profile depicted in FIGS. 7A and 7B of seal member 400 includes seven distinct linear levels or annular steps. Preferably, the longest bristles members are centrally located extending from the center of the substrate 402 to form the tallest step 404 with the shorter steps 406, 408, 410 arranged in descending order on each side of substrate. With this arrangement, seven bands or annular steps of relatively high lateral stiffness will be formed at the levels where the free ends congregate. Each step 404, 406, 408, 410 defines a central aperture with the dimension of the apertures increasing relative to each other toward each of the proximal end distal faces of seal member. This high lateral stiffness will tend to prevent an instrument from slipping radially between the bristles and compromising the fluid-tight seal. Alternatively, steps could be arranged in descending order from one end of the substrate to the other and a seal member may include a different number of steps.

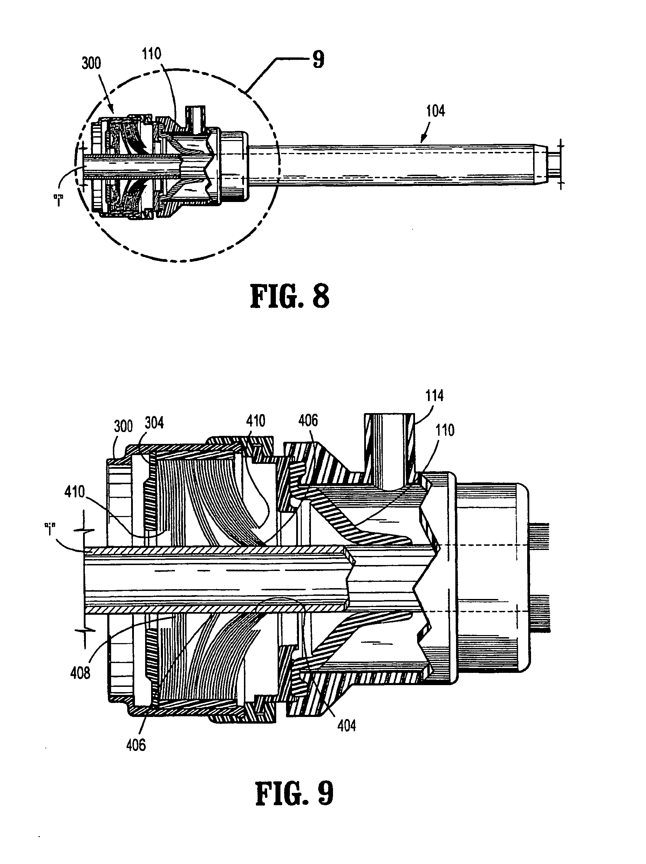

Referring to FIGS. 8 and 9, seal member 400 of FIGS. 7A and 7B is shown mounted to cannula assembly 200. An elongated object such as a surgical instrument, identified generally by reference numeral "i" may be inserted through central aperture 412 of seal assembly 400 and the cannula assembly 200 to perform the desired surgical procedure. As the surgical instrument "i" enters seal assembly 400, the tip of the surgical instrument "i" is engaged by the bristle members of the seal assembly 400. In one arrangement, first (most proximal) step 406 of bristles does not engage the instrument, and the second step 408 of bristles engages the instrument without substantial bending. Steps 410 and 404 are shown as engaging the outer surface of the surgical instrument and pivoting distally to permit passage of the instrument "i". The remaining distal steps 406,408 do not engage the instrument "i", but must pivot to accommodate the pivoting of the adjacent bristles. The second proximal step 408 of bristles provides a robust lateral support for the instrument while steps 410, 404 create the fluid-tight seal. This arrangement is possible due in part to the design of the individual bristles to be relatively strong in buckling when loaded axially when compared to the strength in bending when loaded obliquely. The weakness in bending is desirable because it facilitates the introduction of surgical instruments, while the strength in buckling is desirable it assists in maintaining a central position for the instrument where it can engage sealing bristle members on all sides. Having a step sized such that the bristles forming that step do not bend substantially allows for the axial loading of those bristles by the surgical instrument. The flexibility of the bending bristles permits relatively easy passage of instrument "i" through the seal assembly 400. The ease of instrument passage is further enhanced by the arrangement of the bristle members into a stepped profile because no force is required to pivot the bristle members forming the shortest steps on the proximal end of the seal member, and a reduced force is required to pivot any corresponding steps on the distal end of the seal member which only need to pivot in order to accommodate the displacement of the longer bristles actually forming the fluid-tight seal about the instrument. Instrument "i" is advanced through the cannula assembly 200 whereby the duck-bill seal 208 of the cannula 200 also spreads to allow passage of instrument i. Once positioned within the instrument-seal 400 and cannula assembly 200, surgical instrument "i" may be maneuvered about the internal body cavity.

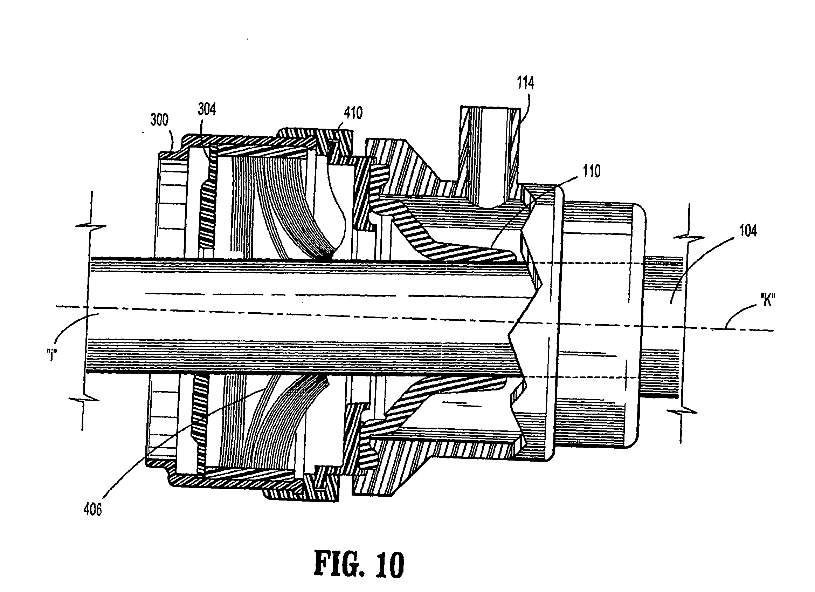

Referring now to FIG. 10, surgical instrument "i" is shown having a lateral offset relative to the central longitudinal axis "k". The seal assembly 100 permits limited unencumbered movement of instrument "i" in a lateral direction (relative to the central longitudinal axis) while maintaining an adequate seal about the instrument. Thus, manipulation of the instrument in any direction, either, longitudinally or radially, will not affect the integrity of the seal, since the resilient material of sealing member 110 will conform to the movements of the instrument and assume a shape necessary to retain a sealing contact with the instrument.

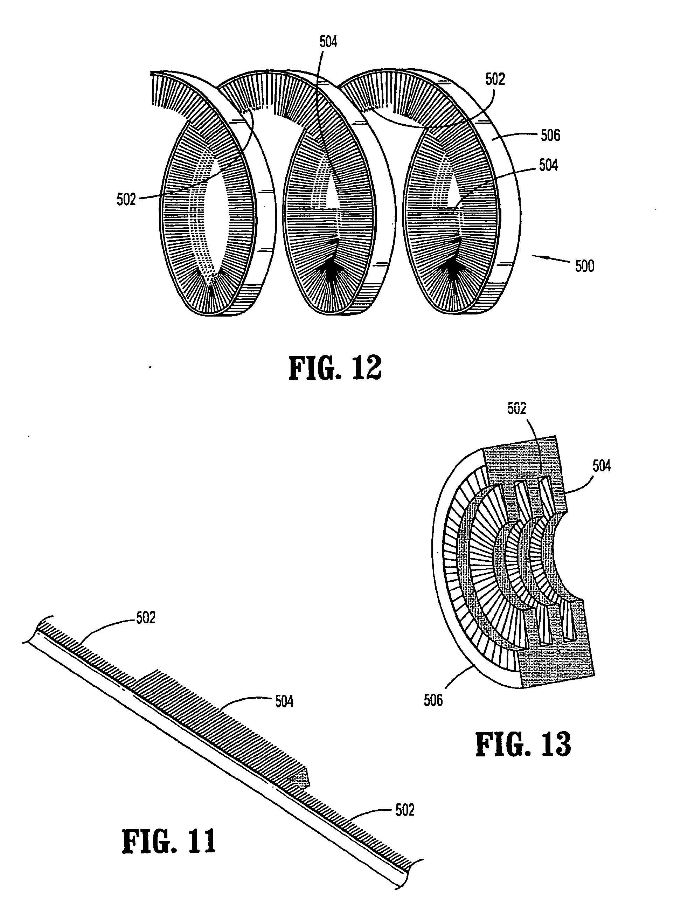

FIGS. 11-13 illustrate another alternate example of a seal member. Seal member 500 illustrates a flat brush design incorporating bristle members 502, 504 having varying lengths extending from substrate 506. In particular, bristle members 502 have a length which is less then the lengths of bristle members 504. In one embodiment, bristle members 504 are centrally located. In the alternative, bristle members 502, 504, may be arranged in alternating manner to define several series of bristle members 502, 504. When substrate 506 is wound for placement in the seal housing, seal member 500 defines a multilayer arrangement of bristle members with varying aperture sizes due to the varied lengths of bristles 502, 504 (see FIG. 13).

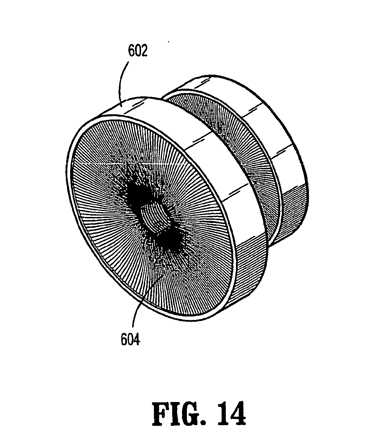

FIG. 14 illustrates an alternate example of the seal member. In accordance with this embodiment, seal member 600 includes substrate 602 and bristle members 604. Bristle members 604 are of constant length. Substrate 602 is arranged such that the outer diameter of the substrate 602 is varied. This arrangement allows for portions of the seal member to expand axially (here the center coil or substrate 602) as the instrument is inserted.

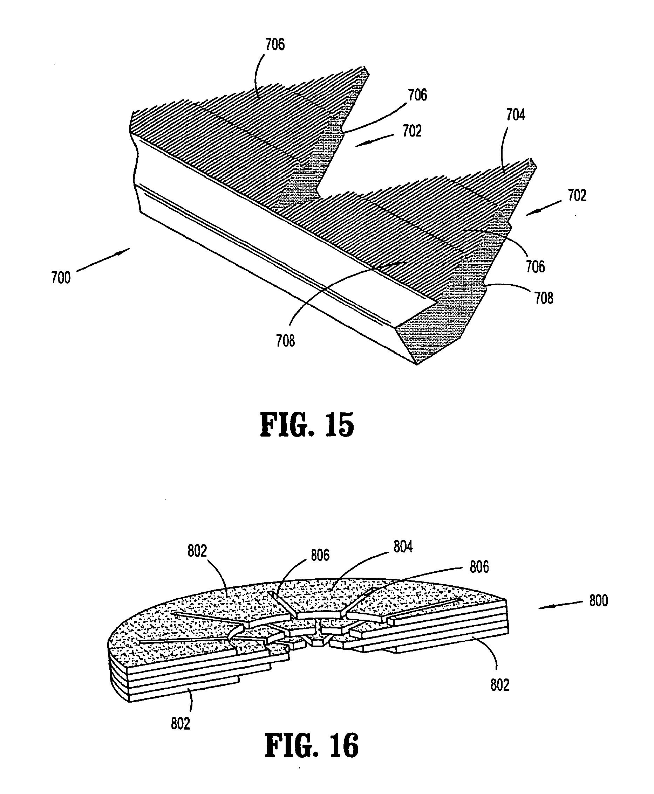

FIG. 15 is another example where seal member 700 has a series of triangular-shaped bristle arrangements 702. Each bristle arrangement 702 includes a plurality of steps having a central located step 704, first lateral steps 706 on each side of the central located step 704 and second lateral steps 708 adjacent respective first lateral steps 706. Seal member 700 formed from this profile is capable of providing high lateral support in predetermined diameters while maintaining a low insertion force.

FIG. 16 shows a seal member 800 created from a stack of substantially flat flexible components 802 having various inner diameters. Each component 802 may have a substrate around its outer circumference where the component is substantially solid. Relatively large, roughly triangular-shaped elements 804 are disposed between a plurality of slits 806 extending through the components. The components are stacked or in superposed relation such that elements 804 and slits 806 are staggered whereby no continuous linear passage extends through seal member 800. Elements 804 may be formed of bristles or alternatively fabricated from a suitable solid elastomeric material.

Although the foregoing disclosure has been described in some detail by way of illustration and example, for purposes of clarity or understanding, it will be obvious that certain changes and modifications may be practiced within the scope of the appended claims.

## Claims

1. A surgical access assembly (10) for sealed reception of an elongated object, which comprises:
an access member (300) having at least one opening (314) configured and dimensioned to permit entry of an elongated object and defining a central longitudinal axis, the access member (300) adapted for positioning within tissue to provide access to an underlying surgical site; and
a seal member (306) mounted relative to the access member (300), the seal member (306) including an outer substrate (326) and a plurality of flexible bristle members (328) extending radially inwardly from the outer substrate (326) toward the central longitudinal axis, the bristle members (328) defining free ends (334) remote from the outer substrate (326) and being adapted to flex and form a substantial seal about the elongated object,
**characterized by**
the free ends (334) of the bristle members (328) having an axial component of direction with respect to the central longitudinal axis in the absence of the elongated object.

2. The surgical access apparatus (10) according to claim 1 therein the bristle members (328) are dimensioned to define a thickness or density which is greater at the free ends (334) thereof relative to remaining portions of the bristle members (328).

3. The surgical access assembly (10) according to any one of the preceding claims wherein the bristle members (328) are arranged to define a varying degree of density of the seal member (306) adjacent the free ends of the bristle members (328) to affect at least one of insertion force or lateral stability of the seal member (306).

4. The surgical access assembly (10) according to any one of the preceding claims wherein the bristle members (328) are arranged to define a bulge (336) adjacent the central longitudinal axis.

5. The surgical access assembly (10) according to any one of the preceding claims wherein the bristle members (328) are arranged to define opposed bulges (336) adjacent the central longitudinal axis.

6. The surgical access assembly (10) according to any one of the preceding claims wherein the bristle members (328) are arranged to define an aperture.

7. The surgical access assembly (10) according to any one of the preceding claims wherein the free ends (334) of the bristle members (328) are arranged to extend in a general axial direction relative to the central longitudinal axis.

## Patentansprüche

1. Chirurgische Zugangsanordnung (10) zur abgedichteten Aufnahme eines verlängerten Gegenstands, umfassend:
ein Zugangsglied (300) mit mindestens einer Öffnung (314), konfiguriert und abgemessen, um den Eintritt eines verlängerten Gegenstands zur möglichen und definierend eine zentrale Längsachse, wobei das Zugangsglied (300) angepasst ist, um innerhalb des Gewebes angeordnet zu werden, um Zugang zu einer darunter liegenden Operationsstelle bereitzustellen; und
ein Dichtungsglied (306), montiert bezüglich des Zugangsglieds (300), wobei das Dichtungsglied (306) ein äußeres Substrat (326) und ein Vielzahl von flexiblen Borstengliedern (328) umfasst, die sich vom äußeren Substrat (326) radial nach innen auf die zentrale Längsachse hin erstrecken, wobei die Borstenglieder (328) freie Enden (34), entfernt vom äußeren Substrat (326) definieren und angepasst sind, um sich zu biegen und eine wesentliche Dichtung um den verlängerten Gegenstand zu bilden,
**dadurch gekennzeichnet, dass**
die freien Enden (334) der Borstenglieder (328) eine axiale Richtungskomponente bezüglich der zentralen Längsachse in Abwesenheit des verlängerten Gegenstands aufweisen.

2. Chirurgische Zugangsanordnung (10) nach Anspruch 1, wobei die Borstenglieder (328) abgemessen sind, um eine Dicke oder Dichte zu definieren, die an den freien Enden (334) davon bezüglich der restlichen Abschnitte der Borstenglieder (328) größer ist.

3. Chirurgische Zugangsanordnung (10) nach einem der vorhergehenden Ansprüche, wobei die Borstenglieder (328) angeordnet sind, um einen verschiednen Grad der Dichte des Dichtungsglieds (306), benachbart den freien Enden der Borstenglieder, zu definieren, um mindestens eine der Einführungskraft oder der seitlichen Stabilität des Dichtungsglieds (306) zu betreffen.

4. Chirurgische Zugangsanordnung (10) nach einem der vorhergehenden Ansprüche, wobei die Borstenglieder (328) angeordnet sind, um eine Wulst (336) zu definieren, benachbart der zentralen Längsachse.

5. Chirurgische Zugangsanordnung (10) nach einem der vorhergehenden Ansprüche, wobei die Borstenglieder (328) angeordnet sind, um gegenüberliegende Wülste (336) zu definieren, benachbart der zentralen Längsachse.

6. Chirurgische Zugangsanordnung (10) nach einem der vorhergehenden Ansprüche, wobei die Borstenglieder (328) angeordnet sind, um eine Öffnung zu definieren.

7. Chirurgische Zugangsanordnung (10) nach einem der vorhergehenden Ansprüche, wobei die freien Enden (334) der Borstenglieder (328) angeordnet sind, um sich in einer allgemeinen axialen Richtung bezüglich der zentralen Längsachse zu erstrecken.

## Revendications

1. Ensemble d'accès chirurgical (10) pour la réception étanche d'un objet allongé, qui comprend :
un élément d'accès (300) ayant au mois une ouverture (314) configurée et dimensionnée pour permettre l'entrée d'un objet allongé et définissant un axe central longitudinal, l'élément d'accès (300) étant adapté pour être positionné dans un tissu de manière à fournir un accès à un site chirurgical sous-jacent ; et
un élément d'étanchéité (306) monté par rapport à l'élément d'accès (300), l'élément d'étanchéité (306) comprenant un substrat externe (326) et une pluralité de soies flexibles (328) s'étendant radialement vers l'intérieur du substrat externe (326) à l'axe central longitudinal, les soies (328) définissant des extrémités libres (334) à distance du substrat externe (326) et étant adaptées pour fléchir et former un joint d'étanchéité substantiel autour de l'objet allongé,
**caractérisé en ce que**
les extrémités libres (334) des soies (328) ont une composante axiale de direction par rapport à l'axe central longitudinal en l'absence de l'objet allongé.

2. Ensemble d'accès chirurgical (10) selon la revendication 1, dans lequel les soies (328) sont dimensionnées pour définir une épaisseur ou une densité qui est supérieure à leurs extrémités libres (334) par rapport aux parties restantes des soies (328).

3. Ensemble d'accès chirurgical (10) selon l'une quelconque des revendications précédentes, dans lequel les soies (328) sont aménagées pour définir un degré variable de densité de l'élément d'étanchéité (306) adjacent aux extrémités libres des soies (328) pour affecter au moins l'une ou l'autre de la force d'insertion et de la stabilité latérale de l'élément d'étanchéité (306).

4. Ensemble d'accès chirurgical (10) selon l'une quelconque des revendications précédentes, dans lequel les soies (328) sont aménagées pour définir un renflement (336) adjacent à l'axe central longitudinal.

5. Ensemble d'accès chirurgical (10) selon l'une quelconque des revendications précédentes, dans lequel les soies (328) sont aménagées pour définir des renflements opposés (336) adjacents à l'axe central longitudinal.

6. Ensemble d'accès chirurgical (10) selon l'une quelconque des revendications précédentes, dans lequel les soies (328) sont aménagées pour définir une ouverture.

7. Ensemble d'accès chirurgical (10) selon l'une quelconque des revendications précédentes, dans lequel les extrémités libres (334) des soies (328) sont aménagées pour s'étendre dans une direction axiale générale par rapport à l'axe central longitudinal.
